# EUROPEAN PATENT APPLICATION

(11) **EP 1 970 053 A1**
(43) Date of publication of application: **17.09.2008**
(21) Application number: 07104157.8
(22) Date of filing: 14.03.2007
(51) Int. Cl.: A61K 9/20

(54) **Pharmaceutical composition**

(71) Applicant: Boehringer Ingelheim Pharma GmbH & Co. KG, 55216 Ingelheim am Rhein (DE)
(72) Inventor: The designation of the inventor has not yet been filed

(57) **Abstract**

A pharmaceutical tablet or tablet layer comprising the angiotensin II receptor antagonist telmisartan in amorpheous form, a basic agent and sorbitol, characterized in that the sorbitol has a specific surface area of between 0.75-3.5 m²/g.

## Description

The present invention relates to a pharmaceutical tablet or tablet layer comprising the active ingredient telmisartan, a basic agent and sorbitol.

Telmisartan is an angiotensin II receptor antagonist developed for the treatment of hypertension and other medical indications as disclosed in EP-A-502314. Its chemical name is 4'-[2-n-propyl-4-methyl-6-(1-methylbenzimidazol-2-yl)-benzimidazol-1-ylmethyl]-biphenyl-2-carboxylic acid having the following structure:

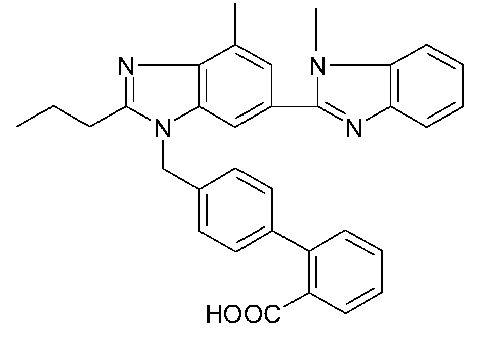

Telmisartan is manufactured and supplied in the free acid form. It is characterized by a very poor solubility in aqueous systems at the physiological pH range of the gastrointestinal tract between pH 1 to 7. As disclosed in WO 00/43370, crystalline telmisartan exists in two polymorphic forms having different melting points. Under the influence of heat and humidity, the lower melting polymorph B transforms irreversibly into the higher melting polymorph A. In addition telmisartan can be prepared in amorpheous form, i.e as a kind of solidified solution or glass having a glass transition temperature Tg of > 50°C or preferably > 80°C.
Telmisartan is commercially available as tables in dosages 20mg, 40mg and 80mg. 20mg tablets are round, measuring 7mm in diameter and about 2.5mm in height. 40mg and 80mg tablets are of oval shapes as are the marketed bilayer tablets containing Hydrochlorothiazide (HCTZ) as a second active ingredient. Lengths of the oval shaped products are between 12 mm and 16.2 mm, width is ranging from 5.8 mm to 7.9 mm and thickness is from 3.8 mm up to 6.2 mm.

The pharmacologic potency of telmisartan necessitates the use of a diluent in a tablet. In addition to the immediate release preparation to be swallowed, it is further desirable to formulate tablets or tablet layers containing Telmisaran in analogy to the description given in the European Pharmacopeia monograph 'Tablets for Use in the Mouth'. As disclosed in WO 03/059327 this means dissolution characteristics determined by an erosion process rather than disintegration of the matrix and requires a suitable water-soluble diluent. WO 03/059327 discloses sorbitol as a preferred diluent for telmisartan and also the registered pharmaceutical compositions of telmisartan comprise sorbitol as a filler. Moreover sorbitol is usually a cheaper excipient than alternative fillers such as xylitol.

Additionally, the poor solubility and lipophilic character of telmisartan requires specific measures to achieve the registered telmisartan dissolution rates from the tablet or tablet layer, respectively. According to the definition of the USP monograph, Q needs to be greater than 75%, preferably greater than 85%. Thus, in accordance with the present invention it has to be ensured that at least 75% and typically at least 85% of the telmisartan drug load are dissolved after 30 min.
According to WO 03/059327 such measures are the presence of a basic agent in the tablet which supports the dissolution of telmisartan in an aqueous medium, and the use of the amorpheous form of telmisartan which can be obtained by spray drying.

Defining D(0.5) as the particle size wherein 50vol.% of the particles of a substance used are below the corresponding numeric D(0.5) value, the particle size D(0.5) of amorpheous telmisartan used to prepare the registered compositions of telmisartan is in the range of 20-55µm while the particle size D(0.5) of sorbitol is in the range of 160-190µm, i.e. beyond 150µm. Smaller particle sizes of sorbitol would improve stability against demixing of the filler sorbitol, the active agent telmisartan and other components of the pharmaceutical composition and would result in an improved and more easily reproducible homogeneity of the pharmaceutical composition. However, sorbitol particle sizes below 150µm do not result in tablets of the required hardness which for the 80mg telmisartan tablets according to example 1 needs to be greater than 100 N, preferably greater than 130 N, determined as crushing strength with an Erweka TBH30 insturment. Expressed as tensile strength as described by Newton et al. (J. Pharm. Pharmac. 23 Suppl., 195S-201S (1971)) for round, flat faced tablets such as the tablet according to example 2, this is equivalent to at least 1.6 N/mm², preferably > 2.1 N/mm² .
A pharmacist knows that demixing of components is a generally observed technical problem if the particle sizes of the active ingredient on the one hand and the diluent on the other hand differ by more than a factor of two as is the case for the registered composition comprising telmisartan and sorbitol (typically 175µm (sorbitol) : 38µm (telmisartan) = 4.6). This considerable size difference eventually gives rise to a composition which has to be dismissed because it does not match the registered technical specifications.

In pharmaceutical production, the specified hardness of tablets is usually achieved through appropriate adjustment of the compaction pressure. Up to the maximum strain allowed for the tooling, this procedure allows compensation for batch to batch variations of compactability, which in turn is caused by natural variations in the physical parameters of the ingredients. For instance, spray dried amorphous telmisartan manufactured according to the method disclosed in WO 03/059327 may have bulk densities between 0.4 and 0.6 g/ml.

Furthermore it is known that compression to higher hardness levels can lead to impaired dissolution. Thus it was previously not possible to consistently obtain tablets or tablet layers comprising telmisartan displaying dissolution rates and hardness levels being both at most preferable levels.

The applicants have now surprisingly found that not the particle size but the specific surface area of the sorbitol used in the preparation of a tablet or tablet layer comprising telmisartan primarily determines the hardness of the tablet or tablet layer and the dissolution rate of the active ingredient telmisartan. Thus, the present invention teaches to use sorbitol with a specific surface area of between 0.75-3.5 m²/g for the preparation of telmisartan tablets or tablet layers while previously the sorbitol used had a specific surface area of between 0.3-0.7 m²/g.

Specific surface area is determined under consideration of the USP monograph <846>, method 2, using a suitable, calibrated BET instrument (Micromeritics ASAP 2400 or equivalent), at 77°K with nitrogen. Sample weight is 3g to 5g. Sample is degassed at 40°C for 2 hours under vacuum. A 6-point determination is made at p/p0 = 0.07 - 0.22.
Tablets manufactured according to the invention display improved hardness as well as higher dissolution rates within the preferred range of compaction pressure of <60% of maximum tooling strength, Further, reproducibility of dissolution and hardness parameters is significantly improved, resulting in both lower intra-batch variation and inter-batch variation. This minimizes the risk of nonconformity to the registered specifications.

The pharmaceutical composition described in the present invention can also be used as a separate tablet layer in a fixed dose combination combining telmisartan with one or more further active ingredients for example in bilayer or trilayer tablets. Examples of such other active ingredients in a separate layer combined with a layer comprising telmisartan are diuretics such as hydrochlorothiazide, calcium receptor antagonists such as amlodipine, ACE inhibitors such as enalapril, lisinopril, ramipril etc, HMG-CoA reductase inhibitors such as simvastatin or atorvastatin, antidiabetic agents such as metformin, glitazones and DPP-IV inhibitors.

Thus, one embodiment of the present invention is a pharmaceutical tablet or tablet layer comprising for example in a dissolving tablet matrix the angiotensin II receptor antagonist telmisartan, a basic agent and sorbitol as a diluent, characterized in that the sorbitol has a specific surface area of between 0.75-3.5 m²/g. A preferred range of the specific surface area of sorbitol is from 1.4-3.0 m²/g and the most preferred range is from 2.0-2.5 m²/g.

Additionally, the sorbitol used for preparations according to the present invention can be characterized by D(0.5) particle sizes in the range of 100-350µm while sizes preferred are in the range of 120-300µm and most preferred sizes are between 150-250µm.

To achieve the required registered dissolution rate of the active agent telmisartan it is preferably used in an amorpheous form with particles of a size less than 80µm, preferably 20-55µm. Such an amorpheous form of telmisartan can be prepared by any suitable method known to those skilled in the art, for instance, by freeze drying of aqueous solutions, coating of carrier particles in a fluidized bed, and solvent deposition on sugar pellets or other carriers. Preferably, however, the substantially amorphous telmisartan is prepared by a spray-drying method as described in WO 03/059327 wherein telmisartan is prepared by dissolving it in purified water with the help of one or more basic agents like sodium hydroxide and/or meglumine. Optionally, a solubilizer and/or a recrystallization retarder may be added. The dry matter content of the starting aqueous solution is generally 10 to 50 wt.%, preferably 20 to 40 wt.%. The aqueous solution is then spray-dried at room temperature or preferably at increased temperatures of, for instance, between 50°C and 100°C in a co-current or countercurrent spray-drier at a spray pressure of, for instance, 1 to 5 bar. Generally speaking, the spray-drying conditions are preferably chosen in such a manner that a spray-dried granulate having a residual humidity of ≤ 5 wt.%, preferably ≤ 3.5 wt.%, is obtained in the separation cyclone. To that end, the outlet air temperature of the spray-drier is preferably kept at a value between about 80°C and 90°C while the other process parameters such as spray pressure, spraying rate, inlet air temperature, etc. are adjusted accordingly. The spray-dried granulate obtained is preferably a fine powder having the following particle size distribution:

| | |
|---|---|
| d₁₀ : | ≤ 20 µm, preferably ≤ 10 µm |
| d₅₀ : | ≤ 80 µm, preferably 20 to 55 µm |
| d₉₀ : | ≤ 350 µm, preferably 50 to 150 µm |

After spray-drying, the active ingredient telmisartan as well as the excipients contained in the spray-dried granulate are in a substantially amorphous state with no crystallinity being detectable. A distinction can be made between low density (0.4 - 0.5 g/ml) and high density (0.5 - 0.6 g/ml) telmisartan granules obtained.

Based on 100 parts by weight of telmisartan, the spray-dried granulate preferably contains 5 to 200 parts by weight of basic agent and, optionally, solubilizer and/or crystallization retarder. Specific examples of suitable basic agents are alkali metal hydroxides such as NaOH and KOH; basic amino acids such as arginine and lysine; and meglumine (N-methyl-D-glucamine), NaOH and meglumine being preferred.

A tablet according to the present invention generally contains between 10 to 160 mg, preferably 20 to 80 mg or 40 to 80 mg, of telmisartan. Preferred dose strengths of telmisartan are 20 mg, 40 mg and 80 mg.

In another embodiment the tablet or tablet layer comprising telmisartan, sorbitol and a basic agent additionally comprises excipients and/or adjuvants such as binders, carriers, disintegrants, fillers, lubricants, flow control agents, crystallization retarders, solubilizers, coloring agents, pH control agents, surfactants and emulsifiers. The excipients and/or adjuvants for the tablet or tablet layer composition are preferably chosen such that a non-acidic, fast dissolving tablet matrix is obtained.

A tablet or tablet layer according to the present invention generally comprises 3 to 50 wt.%, preferably 5 to 35 wt.%, of telmisartan; 0.25 to 20 wt.%, preferably 0.40 to 15 wt.%, of basic agent; and 30 to 95 wt.%, preferably 60 to 80 wt.% of sorbitol.

Mixing is carried out in two stages, i.e. in a first mixing step the spray-dried granulate and the diluent are admixed using , e.g., a high shear mixer or a free fall blender, and in a second mixing step the lubricant is blended with the premix, preferably also under conditions of high shear. Thus, a lubricant such as magnesium stearate is generally added to the premix in an amount of 0.1 to 5 wt.%, preferably 0.3 to 2 wt.%, based on the weight of the tablet or tablet layer composition. The method of the invention is however not limited to these mixing procedures and, generally, alternative mixing procedures may be employed in the various process steps.

Other (optional) constituents may be chosen from one or more of the following excipients and/or adjuvants in the amounts indicated:
10 to 30 wt.%, preferably 15 to 25 wt.%, of binders and carriers;
0.01- 5 wt.% disintegrant;,
0.01 to 5 wt.%, preferably 0.5 to 3 wt.%, of lubricants;
0.01 to 5 wt.%, preferably 0.3 to 2 wt.%, of flow control agents;
0.01 to 20 wt.%, preferably 2 to 8 wt.%, of crystallization retarders;
0.01 to 10 wt.%, preferably 2 to 8 wt.%, of solubilizers;
0.01 to 1.5 wt.%, preferably 0.1 to 0.8 wt.%, of coloring agents;
0.01 to 10 wt.%, preferably 2 to 8 wt.%, of pH control agents;
0.01 to 5 wt.%, preferably 0.05 to 1 wt.%, of surfactants and emulsifiers.

The tablets of the present invention are slightly hygroscopic and therefore preferably packaged using a moisture-proof packaging material such as aluminium foil blister packs, or polypropylene tubes and HDPE bottles which preferably contain a desiccant.

A preferred method of producing a tablet or tablet layer according to the present invention comprises
a) preparing an aqueous solution of telmisartan, at least one basic agent and, optionally, a solubilizer and/or a crystallization retarder;
b) spray-drying said aqueous solution to obtain a spray-dried granulate;
c) mixing said spray-dried granulate with a sorbitol having a specific surface area of between 0.75-3.5 m²/g to obtain a premix;
d) mixing said premix with a lubricant to obtain a final blend;
e) optionally, adding other excipients and/or adjuvants in any of steps a) to d); and
f) compressing said final blend to a tablet or tablet layer.

Said method can be used for the manufacture of a tablet or tablet layer according to the present invention to treat hypertension either alone or in combination with the treatment or prevention of a condition selected from the group consisting of chronic stable angina, vasospastic angina, stroke, myocardial infarction, transient ischemic attack, congestive heart failure, cardiovascular disease, diabetes, insulin resistance, impaired glucose tolerance, pre-diabetes, type 2 diabetes mellitus, diabetic nephropathy, metabolic syndrome (syndrome X), obesity, dyslipidemia, hypertriglyceridemia, elevated serum concentrations of C-reactive protein, elevated serum concentrations of lipoprotein(a), elevated serum concentration of homocysteine, elevated serum concentration of low-density lipoprotein (LDL)-cholesterol, elevated serum concentration of lipoprotein-associated phospholipase (A2), reduced serum concentration of high density lipoprotein (HDL)-cholesterol, reduced serum concentration of HDL(2b)-cholesterol, reduced serum concentration of adiponectin, cognitive decline and dementia.

Particularly preferred is the additional treatment or prevention of chronic stable angina, vasospastic angina, stroke, myocardial infarction, congestive heart failure, diabetes, dyslipidemia or dementia.

Finally, a tablet layer according to the present invention can be compressed with one or more tablet layer compositions comprising other active ingredients into multiple layer tablets such as bi- and trilayer tablets of the target tablet weight with appropriate size and crushing strength, using an appropriate tablet press. Optional an appropriate external lubricant spray system for the dies and punches can be used during manufacturing of these tablets in order to improve lubrication.

In order to further illustrate the present invention, the following non-limiting examples are given.

### EXAMPLES

| Example 1: **Tablet (layer) comprising 80 mg telmisartan** | | |
|---|---|---|
| **Constituents** | **mg per tablet** | **% of Telmisartan layer** |
| Telmisartan | 80.000 | 16.667 |
| Sodium hydroxide | 6.720 | 1.400 |
| Povidone | 24.000 | 5.000 |
| Meglumine | 24.000 | 5.000 |
| Sorbitol 2m²/g | 337.280 | 70.267 |
| Magnesium stearate | 8.000 | 1.667 |
| Purified water * | * | * |
| **Total Telmisartan layer** | **480.000** | **100.000** |

| | | |
|---|---|---|
| * Volatile component, does not remain in final product | | |

| Example 2: **Tablet (layer) comprising 40 mg telmisartan** | | |
|---|---|---|
| **Constituents** | **mg per tablet** | **% of Telmisartan layer** |
| Telmisartan | 40.000 | 16.667 |
| Sodium hydroxide | 3.360 | 1.400 |
| Povidone | 12.000 | 5.000 |
| Meglumine | 12.000 | 5.000 |
| Sorbitol 2m²/g | 168.640 | 70.267 |
| Magnesium stearate | 4.000 | 1.667 |
| Purified water * | * | * |
| **Total Telmisartan layer** | **240.000** | **100.000** |

| | | |
|---|---|---|
| * Volatile component, does not remain in final product | | |

| Example 3: **Tablet (layer) comprising 20 mg telmisartan** | | |
|---|---|---|
| **Constituents** | **mg per tablet** | **% of Telmisartan layer** |
| Telmisartan | 20.000 | 16.667 |
| Sodium hydroxide | 1.680 | 1.400 |
| Povidone | 6.000 | 5.000 |
| Meglumine | 6.000 | 5.000 |
| Sorbitol (2 m²/g) | 84.320 | 70.267 |
| Magnesium stearate | 2.000 | 1.667 |
| Purified water * | * | * |
| **Total Telmisartan layer** | **120.000** | **100.000** |

| | | |
|---|---|---|
| * Volatile component, does not remain in final product | | |

| Example 4: **80 mg telmisartan and 12.5 mg hydrochlorothiazide (HCTZ) bilayer tablet** | | | |
|---|---|---|---|
| **Constituents** | **mg per tablet** | **% of Telmisartan layer** | **% of HCTZ layer** |
| Telmisartan | 80.000 | 16.667 | |
| Sodium hydroxide | 6.720 | 1.400 | |
| Povidone | 24.000 | 5.000 | |
| Meglumine | 24.000 | 5.000 | |
| Sorbitol 2m²/g | 337.280 | 70.267 | |
| Magnesium stearate | 8.000 | 1.667 | |
| Purified water * | * | * | |
| **Total Telmisartan layer** | **480.000** | **100.000** | |
| Hydrochlorothiazide | 12.500 | | 6.250 |
| Lactose monohydrate | 112.170 | | 56.085 |
| Microcrystalline cellulose | 64.000 | | 32.000 |
| Iron oxide red | 0.330 | | 0.165 |
| Sodium starch glycolate | 4.000 | | 2.000 |
| Maize starch dried | 6.000 | | 3.000 |
| Magnesium stearate | 1.000 | | 0.500 |
| **Total HCTZ layer** | **200.000** | | **100.000** |
| | | | |
| **Total bilayer tablet** | **680.000** | | |

| | | | |
|---|---|---|---|
| * Volatile component, does not remain in final product | | | |

| Example 5: **Comparison of 80mg telmisartan tablet (layer) hardness** Tablet shape oval, 16.2mm x 7.9mm x 4.6mm (L x W x H) | | | | | | |
|---|---|---|---|---|---|---|
| **a. Sorbitol with specific surface area 0.5 - 0.7 m²/g used** | | | | | | |
| **Telmisartan density group** | **Tablet batch** | **Hardness** **(N)** | **Compaction force** **(kN)** | **Compaction pressure** **(MPa)** | **Ratio Compaction pressure/Hardness** **(MPa/N)** | |
| LD* | 204348 | 122 | 21 | 194 | 1.6 | |
| | 204349 | 114 | 32 | 295 | 2.6 | |
| | 204350 | 114 | 32 | 295 | 2.6 | |
| HD** | 508489 | 109 | 24 | 222 | 2.0 | |
| | 508490 | 109 | 25 | 231 | 2.1 | |
| | 508491 | 122 | 28 | 259 | 2.1 | |
| | 510280 | 107 | 30 | 277 | 2.6 | |
| | 602146 | 79 | 38 | 351 | 4.4 | |
| | 602147 | 90 | 38 | 351 | 3.9 | |
| | | 107 | 30 | 275 | 2.7 | **mean RSD (%) minimal maximal** |
| | | 13 | 20 | 20 | 35 | |
| | | 79 | 21 | 194 | 1.6 | |
| | | 122 | 38 | 351 | 4.4 | |

| **b. Sorbitol with specific surface area 2 m²/g used** | | | | | | |
|---|---|---|---|---|---|---|
| **Telmisartan density group** | **Tablet batch** | **Hardness** **(N)** | **Compaction force** **(kN)** | **Compaction pressure** **(MPa)** | **Ratio Compaction pressure/Hardness** **(MPa/N)** | |
| LD* | #75 | 222 | 20 | 185 | 0.8 | |
| HD** | #72 | 216 | 20 | 185 | 0.9 | |
| | #73 | 211 | 20 | 185 | 0.9 | |
| | #74 | 215 | 20 | 185 | 0.9 | |
| | 606035 | 190 | 20 | 185 | 1.0 | |
| | 606036 | 189 | 20 | 185 | 1.0 | |
| | 606037 | 191 | 20 | 185 | 1.0 | |
| | 702619 | 198 | 24 | 222 | 1.1 | |
| | | 204 | 21 | 189 | 0.9 | **mean RSD (%) minimal maximal** |
| | | 7 | 7 | 7 | 1.0 | |
| | | 189 | 20 | 185 | 0.8 | |
| | | 222 | 24 | 222 | 1.1 | |

| | | | | | | |
|---|---|---|---|---|---|---|
| * LD = low density 0.4 - 0.5 g/ml ** HD = high density 0.5 - 0.6 g/ml | | | | | | |

| Example 6: **Comparison of 80 mg telmisartan tablet (layer) dissolution rate** | | | | | |
|---|---|---|---|---|---|
| **a. Sorbitol with specific surface area 0.5 - 0.7 m²/g used** | | | | | |
| **Telmisartan density group** | **Tablet batch** | **Dissolution mean value** **(%)** | **Dissolution single minimal value** **(%)** | **Variation of Dissolution (mean-minimal) value** **(%)** | |
| LD | 204348 | 88 | 83 | 5 | |
| | 204349 | 82 | 74 | 8 | |
| | 204350 | 83 | 78 | 5 | |
| HD | 508489 | 88 | 76 | 12 | |
| | 508490 | 86 | 82 | 4 | |
| | 508491 | 91 | 79 | 12 | |
| | 510280 | 91 | 85 | 6 | |
| | 602146 | 88 | 81 | 7 | |
| | 602147 | 85 | 72 | 13 | |
| | | 87 | 79 | 8 | **mean minimal maximal ≤ 75 (n)** |
| | | 82 | 72 | 4 | |
| | | 91 | 85 | 12 | |
| | | | 2 | | |

| **b. Sorbitol with specific surface area 2 m²/g used** | | | | | |
|---|---|---|---|---|---|
| **Telmisartan density group** | **Tablet batch** | **Dissolution mean value** **(%)** | **Dissolution single minimal value** **(%)** | **Variation of Dissolution (mean-minimal) value** **(%)** | |
| LD | #75 | 95 | 95 | 0 | |
| HD | #72 | 93 | 84 | 9 | |
| | #73 | 95 | 91 | 4 | |
| | #74 | 94 | 93 | 1 | |
| | 606035 | 87 | 84 | 3 | |
| | 606036 | 84 | 78 | 6 | |
| | 606037 | 87 | 83 | 4 | |
| | 702619 | 94 | 93 | 1 | |
| | | 91 | 87 | 4 | **mean minimal maximal ≤ 75 (n)** |
| | | 84 | 78 | 0 | |
| | | 95 | 95 | 9 | |
| | | | 0 | | |

| Example 7: **Comparison of 20mg telmisartan tablet (layer) hardness** Tablet shape round, diameter 7 mm, thickness 2.5 mm | | | | | | | | |
|---|---|---|---|---|---|---|---|---|
| **a. Sorbitol with specific surface area 0.5 - 0.7 m²/g used** | | | | | | | | |
| **Telmisartan density group** | **Tablet batch** | **Hardness** **(N)** | **Compaction force** **(kN)** | **Tensile strength** **(N/mm²)** | **Compaction pressure** **(MPa)** | **Ratio Compaction pressure /** | | |
| | | | | | | **Hardness** **(MPa/N)** | **Tensile strength** | |
| HD | 509997 | 58 | 11 | 2.1 | 286 | 4.9 | 135 | |
| | 509998 | 56 | 11 | 2.0 | 286 | 5.1 | 140 | |
| | 509999 | 59 | 10 | 2.1 | 260 | 4.4 | 121 | |
| | 602786 | 72 | 11 | 2.6 | 286 | 4.0 | 109 | |
| | 603693 | 52 | 10 | 1.9 | 260 | 5.0 | 137 | |
| | 603733 | 71 | 12 | 2.6 | 312 | 4.4 | 121 | |
| | 604178 | 47 | 22 | 1.7 | 571 | 12.2 | 334 | |
| | | 59 | 12 | 2.2 | 323 | 5.7 | 157 | **mean** |

| **b. Sorbitol with specific surface area 2 m²/g used** | | | | | | | | |
|---|---|---|---|---|---|---|---|---|
| **Telmisartan density group** | **Tablet batch** | **Hardness** **(N)** | **Compaction force** **(kN)** | **Tensile strength** **(N/mm²)** | **Compaction pressure** **(MPa)** | **Ratio Compaction pressure /** | | |
| | | | | | | **Hardness** **(MPa/N)** | **Tensile strength** | |
| HD | 702914 | 67 | 9.5 | 2.4 | 247 | 3.7 | 101 | |

| Example 8: **Comparison of telmisartan tablet layer hardness** (80 mg telmisartan plus 12.5 mg hydrochlorothiazide bilayer tablet) | | | | | | |
|---|---|---|---|---|---|---|
| **a. Sorbitol with specific surface area 0.5 - 0.7 m²/g used** | | | | | | |
| **Telmisartan density group** | **Tablet batch** | **Hardness** **(N)** | **Compaction force** **(kN)** | **Compaction pressure** **(MPa)** | **Ratio Compaction pressure/Hardness** **(Mpa/N)** | |
| HD | 506918 | 133 | 16 | 148 | 1.1 | |
| | 506919 | 135 | 16 | 148 | 1.1 | |
| | 506920 | 131 | 15 | 139 | 1.1 | |
| | 509994 | 129 | 19 | 175 | 1.4 | |
| | 509995 | 127 | 19 | 175 | 1.4 | |
| | 509996 | 125 | 17 | 157 | 1.3 | |
| | | 130 | 17 | 157 | 1.2 | **Mean** |

| **b. Sorbitol with specific surface area 2 m²/g used** | | | | | | |
|---|---|---|---|---|---|---|
| **Telmisartan density group** | **Tablet batch** | **Hardness** **(N)** | **Compaction force** **(kN)** | **Compaction pressure** **(MPa)** | **Ratio Compaction pressure/Hardness** **(Mpa/N)** | |
| HD | 609031 | 180 | 14 | 129 | 0.7 | |

| Example 9: **Comparison of telmisartan dissolution rate** (80 mg telmisartan plus 12.5 mg hydrochlorothiazide bilayer tablet) | | | | | |
|---|---|---|---|---|---|
| **a. Sorbitol with specific surface area 0.5 - 0.7 m²/g used** | | | | | |
| **Telmisartan density group** | **Tablet batch** | **Dissolution mean value** **(%)** | **Dissolution single minimal value** **(%)** | **Variation of Dissolution (mean-minimal) value** **(%)** | |
| HD | 506918 | 102 | 99 | 3 | |
| | 506919 | 94 | 92 | 2 | |
| | 506920 | 100 | 97 | 3 | |
| | 509994 | 97 | 95 | 2 | |
| | 509995 | 94 | 88 | 6 | |
| | 509996 | 98 | 96 | 2 | |
| | | 98 | 94 | 3 | **mean minimal maximal** |
| | | 94 | 88 | 2 | |
| | | 102 | 99 | 6 | |

| **b. Sorbitol with specific surface area 2 m²/g used** | | | | | |
|---|---|---|---|---|---|
| **Telmisartan density group** | **Tablet batch** | **Dissolution mean value** **(%)** | **Dissolution single minimal value** **(%)** | **Variation of Dissolution (mean-minimal) value** **(%)** | |
| HD | 609031 | 97 | 94 | 3 | |

## Claims

1. A pharmaceutical tablet or tablet layer comprising the angiotensin II receptor antagonist telmisartan in amorpheous form, a basic agent and sorbitol, **characterized in that** the sorbitol has a specific surface area of between 0.75-3.5 m²/g, preferably 1.4-3.0 m²/g and most preferred 2.0-2.5 m²/g.

2. The tablet or tablet layer of claim 1 comprising sorbitol having a D(0.5) average particle size of 100-350µm, preferably 120-300µm and most preferably 150-200µm.

3. The tablet or tablet layer of claim 1, wherein the basic agent is selected from alkali metal hydroxides, basic amino acids and meglumine.

4. The tablet or talet layer of claim 1 containing 10-160 mg, preferably 20-80 mg or 40-80 mg telmisartan.

5. The tablet or tablet layer of claim 1 comprising amorphous telmisartan having an average particle size of <80µm, preferably 20-55µm.

6. The tablet or tablet layer of claim 1 additionally comprising other excipients or adjuvants.

7. The tablet or tablet layer of claim 6, wherein the other excipients and adjuvants are selected from binders, carriers, disinterants, fillers, lubricants, flow control agents, crystallization retarders, solubilizers, coloring agents, pH control agents, surfactants and emulsifiers.

8. The tablet or tablet layer of claim 1 produced by spray-drying an aqueous solution comprising telmisartan and a basic agent to obtain a spray-dried granulate, mixing said spray-dried granulate with the sorbitol to obtain a premix and mixing said premix with a lubricant to obtain a final blend.

9. The tablet or tablet layer of claim 1 packaged in a moisture proof packaging material such as aluminium foil blister packs, or polypropylene tubes and HDPE bottles.

10. A method for the manufacture of a tablet or tablet layer of claim 1 to treat hypertension either alone or in combination with the treatment or prevention of a condition selected from the group consisting of chronic stable angina, vasospastic angina, stroke, myocardial infarction, transient ischemic attack, congestive heart failure, cardiovascular disease, diabetes, insulin resistance, impaired glucose tolerance, pre-diabetes, type 2 diabetes mellitus, diabetic nephropathy, metabolic syndrome (syndrome X), obesity, dyslipidemia, hypertriglyceridemia, elevated serum concentrations of C-reactive protein, elevated serum concentrations of lipoprotein(a), elevated serum concentration of homocysteine, elevated serum concentration of low-density lipoprotein (LDL)-cholesterol, elevated serum concentration of lipoprotein-associated phospholipase (A2), reduced serum concentration of high density lipoprotein (HDL)-cholesterol, reduced serum concentration of HDL(2b)-cholesterol, reduced serum concentration of adiponectin, cognitive decline and dementia.

11. The method of claim 10 wherein the condition treated or prevented is chronic stable angina, vasospastic angina, stroke, myocardial infarction, congestive heart failure, diabetes, dyslipidemia or dementia.

12. A process for the preparation of the pharmaceutical tablet or tablet layer of claim 1 comprising
spray-drying an aqueous solution comprising telmisartan and a basic agent to obtain a spray-dried granulate,
mixing said spray-dried granulate with the sorbitol having a specific surface area; of between 0.75-3.5 m²/g to obtain a premix;
mixing said premix with a lubricant to obtain a final blend; and
compressing said final blend to a tablet or tablet layer.
